# EUROPÄISCHE PATENTANMELDUNG

(11) **EP 4 414 942 A1**
(43) Veröffentlichungstag der Anmeldung: **14.08.2024**
(21) Anmeldenummer: 24156027.5
(22) Anmeldetag: 06.02.2024
(51) Int. Cl.: G06V 10/14, G01N 33/42, G06V 10/10, G06V 10/24, G06V 10/26, G06V 10/44, G06V 10/48, G06V 10/762, G06V 10/82, G06V 20/52, G06V 20/64

(54) **AUTOMATISIERTE BOHRKERNVERMESSUNG**

(30) Priorität: 08.02.2023 DE 102023103084
(71) Anmelder: infraTest Prüftechnik GmbH, 74336 Brackenheim-Botenheim (DE)
(72) Erfinder: Peiker, Mario, Ernst a. d. Mosel (DE)
(74) Vertreter: Durm Patentanwälte PartG mbB

(57) **Zusammenfassung**

Die vorliegende Erfindung betrifft ein Verfahren zum Vermessen eines Bohrkerns (12) eines Straßenbelags umfassend die Schritte: In Drehung Versetzen (S10) des Bohrkerns (12); Beleuchten (S20) des Bohrkerns (12) mit einer Beleuchtungseinheit (16); Aufnehmen (S30) des in Drehung versetzten und beleuchteten Bohrkerns (12) mit einer Kameraeinheit (18); Analysieren (S40) des aufgenommenen Bohrkerns (12) bezüglich seiner Beschaffenheit, wobei der Bohrkern (12) mittels eines Analysealgorithmus automatisiert bezüglich eines der folgenden Parameter analysiert wird: Farbe des Bitumen-Mineralstoffgemischs, Korngrößen, Kornverteilung, Porigkeit, Anzahl der Schichten, Kompaktierung der Schichten, Schichtgrenzen (30), Hohlräume und/oder Farbübergänge zwischen den Schichten. Die vorliegende Erfindung betrifft ferner eine entsprechende Vorrichtung.

## Beschreibung

Die vorliegende Erfindung betrifft ein Verfahren zum Vermessen eines Bohrkerns eines Straßenbelags sowie eine entsprechende Vorrichtung.

Straßen werden üblicherweise anhand der zu erwartenden Verkehrsbeanspruchung dimensioniert. Zur Überprüfung einer anstehenden Sanierung oder zur Kontrolle der Bauleistung ist es bekannt, Bohrkerne zu entnehmen. Diese Bohrkerne werden mithilfe eines Straßenkernbohrgeräts in regelmäßigen Abständen entnommen. Bei Kontrollprüfungen werden die entnommenen Bohrkerne hinsichtlich optischer Auffälligkeiten, insbesondere der Schicht- bzw. Einbaudicke oder Einbaumenge und des Schichtverbunds der Einzellagen geprüft. Diese Prüfung erfolgt derzeit manuell durch geschulte Prüfer, die mit einem Stahllineal angelegt an die Oberfläche der Bohrkerne an verschiedenen Punkten die Dimensionierung der Schichten messen.

Diese Vorgehensweise ist personal-, kosten- und zeitintensiv.

Die vorliegende Erfindung stellt sich die Aufgabe, eine Möglichkeit anzugeben, Bohrkerne, insbesondere Bohrkerne eines Straßenbelags, verbessert zu untersuchen. Vorzugsweise soll eine effiziente automatisierte Möglichkeit geschaffen werden, Bohrkerne zuverlässig vorzugsweise optisch zu vermessen und zu analysieren.

Gelöst wird diese Aufgabe durch ein Verfahren zum Vermessen eines Bohrkerns eines Straßenbelags, umfassend die Schritte:
in Drehung Versetzen des Bohrkerns;
Beleuchten des Bohrkerns mit einer Beleuchtungseinheit;
Aufnehmen des in Drehung versetzten und beleuchteten Bohrkerns mit einer Kameraeinheit, die vorzugsweise eine einzige Kamera oder mehrere Kameras umfassen kann; und
Analysieren des aufgenommenen Bohrkerns bezüglich seiner Beschaffenheit; wobei der Bohrkern mittels eines Analysealgorithmus automatisiert bezüglich wenigstens eines der folgenden Parameter analysiert wird:
   Farbe des Bitumen-Mineralstoffgemischs, Korngrößen, Kornverteilung, Porigkeit, Anzahl der Schichten, Kompaktierung der Schichten, Schichtgrenzen, Hohlräume und/oder Farbübergänge zwischen den Schichten.

Ferner wird die Aufgabe gelöst durch eine Vorrichtung zum Vermessen eines Bohrkerns eines Straßenbelags umfassend: einen Rollenstand, vorzugsweise mit Schrittmotorantrieb, zum in Drehung Versetzen des auf dem Rollenstand angeordneten Bohrkerns;
eine Beleuchtungseinheit, vorzugsweise in Form einer dimmbaren Fotokammer mit kippbarer LED-Flächenbeleuchtung, zum Beleuchten des Bohrkerns; eine Kameraeinheit zum Aufnehmen des in Drehung versetzten und beleuchteten Bohrkerns, vorzugsweise für Bohrkerne mit einem Durchmesser bis zu 200 mm und einer Länge bis zu 650 mm.

Die Vorrichtung umfasst ferner eine Analyseeinheit, die dazu ausgebildet ist, Schichtgrenzen des Bohrkerns automatisiert zu erfassen und vorzugsweise ein Verfahren wie zuvor definiert durchzuführen.

Die Vorrichtung umfasst vorzugsweise zumindest eine Kamera, ein Bildverarbeitungssystem mit Objektiv und ist zur Analyse und Vermessung der Schichten des Bohrkerns ausgebildet, insbesondere zur Erkennung von Vertiefungen, Poren und Farben auf der Bohrkernoberfläche. Hierbei kann eine speziell adaptierte Kamera zum Einsatz kommen. Durch das in Drehung Versetzen des Bohrkerns und Beleuchten des Bohrkerns kann die Oberfläche des Bohrkerns schnell und zuverlässig von der Kameraeinheit erfasst werden. Insbesondere ist es denkbar, den Rollenstand mit einem Auslöser der Kameraeinheit zu koppeln, sodass ein Ausmaß der Rotation des Bohrkerns zwischen zwei Kameraaufnahmen exakt bekannt ist und bei der Analyse entsprechend berücksichtigt werden kann. Das Analysieren mittels eines Algorithmus, insbesondere eines Analysealgorithmus, ermöglicht vergleichbare Ergebnisse der ermittelten Parameter. Insbesondere ist es denkbar, den Analysealgorithmus lernend auszubilden, sodass mit jeder zusätzlichen Analyse eine Verbesserung der Analysefähigkeit eintreten kann.

Es versteht sich, dass ein Bohrkern auch abschnittsweise vermessen werden kann. Insbesondere kann es bei älteren Straßenbelägen vorkommen, dass ein Bohrkern bei der Entnahme bricht. Hierbei können die einzelnen Bohrkernbruchstücke aufgenommen und anschließend wieder zu einem Gesamtbild des Bohrkerns zusammengefügt werden. Alternativ können auch jeweils die einzelnen Bohrkernbruchstücke untersucht werden.

Bevorzugte Ausgestaltungen der Erfindung werden in den abhängigen Ansprüchen beschrieben. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Insbesondere kann die Vorrichtung entsprechend der für das Verfahren in den abhängigen Ansprüchen beschriebenen Ausgestaltungen ausgeführt sein.

In bevorzugter Ausführungsform umfasst der Analysealgorithmus eine künstliche Intelligenz. Besonders bevorzugt umfasst der Analysealgorithmus ein neuronales Netz, das insbesondere auf einem residualen Netzwerk basiert. Das neuronale Netz kann mit mindestens drei Schichten, bevorzugt mit mindestens zehn Schichten, besonders bevorzugt mit mehr als fünfzehn Schichten ausgebildet sein. Insbesondere kann das neuronale Netz mit achtzehn Schichten oder mehr konfiguriert sein. Zur Verarbeitung mittels der künstlichen Intelligenz werden Einzelaufnahmen des Bohrkerns zu einem durchgängigen Bild zusammengefügt. Hierbei können Verzerrungen ausgeglichen und Überschneidungen abgeschnitten werden, sodass ein flaches unverzerrtes Abbild der Manteloberfläche des Bohrkerns erhalten wird. Dieses Bild kann von einer künstlichen Intelligenz, insbesondere einem neuronalen Netzwerk verarbeitet werden, wobei das neuronale Netz alle Pixel markiert, die einer Schichtgrenze zuzuordnen sind. Durch die Verarbeitung mittels eines neuronalen Netzes kann folglich ein Binärbild erhalten werden, das die zwei Informationen, nämlich Schichtgrenze oder Hintergrund, anzeigt.

Vorteilhafterweise wird das Beleuchten des Bohrkerns mittels einer Beleuchtungseinheit mit zwei Flächenlichtern, die parallel zur Lage des Bohrkerns angebracht sind, durchgeführt. Hierbei erfolgt bevorzugt eine Beleuchtung von oben, also im Wesentlichen von der gleichen Seite, von der auch die Kameraaufnahme stattfindet. Unter Flächenlichtern sind insbesondere flächig ausgedehnte, vorzugsweise isotrop strahlende, Leuchtkörper zu verstehen. Insbesondere können die Flächenlichter als LED-Flächen ausgebildet sein. Hierdurch kann ein sogenanntes direktes Auflicht erhalten werden, wobei die Kameraeinheit bevorzugt zwischen den beiden Flächenlichtern angeordnet ist. Durch die Verwendung von wenigstens zwei Flächenlichtern kann eine optimale Ausleuchtung des Bohrkerns zur Aufnahme mittels der Kameraeinheit erreicht werden.

In einer bevorzugten Ausführungsform wird der Bohrkern während der Rotation mit weißem Licht beleuchtet. Bevorzugt kann der Bohrkern alternativ oder zusätzlich mit rotem, blauem und/oder grünem Licht abwechselnd beleuchtet werden. Ergänzend oder alternativ kann der Bohrkern besonders bevorzugt mit polarisiertem Licht beleuchtet werden. Es versteht sich, dass auch eine Kombination der Vorgenannten möglich ist. Es versteht sich, dass auch weitere Lichtfarben bzw. Wellenlängen zur Vermessung des Bohrkerns denkbar sind. Insbesondere können alle Wellenlängen zur Vermessung des Bohrkerns verwendet werden, die von einer entsprechenden Kamera erfassbar sind. Durch das Beleuchten des Bohrkerns mit Licht mit verschiedenen Wellenlängen können verschiedene und insbesondere vorteilhafte Lichtreflektionen bei der Aufnahme des Bohrkerns erhalten werden. Diese Lichtreflektionen können die nachfolgende Bildverarbeitung erleichtern.

Asphaltoberflächen weisen spezifische Materialeigenschaften und Strukturen auf, die die Art und Weise beeinflussen, wie diese Oberflächen das einfallende Licht reflektieren. Die Lichtreflektion variiert abhängig von der Textur des Asphalts, seiner Rauheit und/oder Oberflächenbeschaffenheit.

In besonders bevorzugter Ausführungsform ist wenigstens eine Kamera der Kameraeinheit mit einem Polarisationsfilter ausgestattet, der spezifische Ausrichtungen des polarisierten Lichts filtert. Hierbei können Details oder Unterschiede auf der Oberfläche des Asphalts, die durch die Polarisationseigenschaften des reflektierten Lichts entstehen, verbessert erfasst werden. Bei einer Analyse der Information des polarisierten Lichts können bestimmte Merkmale des Asphalts sichtbar gemacht werden, die bei herkömmlichen Aufnahmen möglicherweise schwer oder gar nicht erkennbar sind. Insbesondere umfassen diese Merkmale Risse, Unebenheiten oder spezifische Strukturen, die durch unterschiedliche Reflexionseigenschaften des polarisierten Lichts hervorgehoben oder erst erkennbar gemacht werden können.

Vorteilhafterweise erfolgt ein Entzerren und Zuschneiden der Einzelaufnahmen des Bohrkerns durch Anwendung von Transformationsmatrizen. Insbesondere können hierbei 3x3 projektive Transformationsmatrizen Anwendung finden. Bei der Anwendung der Transformationsmatrizen werden Einzelaufnahmen zunächst entzerrt und zugeschnitten, sodass zwischen den Aufnahmen keine Überlappungsbereiche mehr sichtbar sind. Anschließend werden die Einzelaufnahmen nebeneinander in gerader Linie angeordnet, sodass ein durchgängiges Bild entsteht, bei dem vorzugsweise die Überlappungsbereiche entfernt sind. Mit anderen Worten kann ein flaches Abbild der Manteloberfläche des Bohrkerns erfasst werden. Es werden also zunächst einzelne Aufnahmen angefertigt und über wenigstens eine Transformationsmatrix so verändert, dass sie exakt zueinander positioniert sind. Die Kanten der Aufnahmen können pixelgenau zueinander positioniert werden, um ein realistisches, durchgehendes Abbild, also ein Gesamtbild, der Objektebene bereitzustellen, um die Manteloberfläche verzerrungsfrei darzustellen. Es versteht sich, dass bei einer schrittweisen Abtastung mit einer einzelnen Kamera die so erhaltenen Einzelbilder auf die oben genannte Weise zusammengeführt werden können. Alternativ können auch mehrere Kameras zeitgleich verwendet werden, um die Einzelbilder in kürzerer Zeit aufzunehmen.

Bevorzugt wird ein Überlappungsbereich erfasst. Unter Überlappungsbereich ist insbesondere ein Bereich des Bohrkehrs zu verstehen, der aus zwei verschiedenen Kamerapositionen erfasst wird. Basierend auf der Erfassung des Überlappungsbereichs wird eine topografische Information des Bohrkerns errechnet, die die Erhebungen und Vertiefungen in der Objektebene, also an der Manteloberfläche des Bohrkerns sichtbar macht. Da die Position der Kamera bekannt ist, kann ein Stereobildprinzip genutzt werden, sodass durch Rektifizierung Erhebungen in der Objektebene in die Bildtiefe errechnet werden können. Durch dieses Vorgehen kann der Informationsgewinn bei der Bohrkernvermessung weiter erhöht werden. Insbesondere ist es denkbar, die Vertiefungen und/oder Erhebungen in der Oberfläche des Bohrkerns als 3D-Relief zu erfassen. Besonders bevorzugt werden hierfür hochauflösende Sensoren bzw. Kameras verwendet. Es versteht sich, dass hierfür auch mehrere Kameras verwendet werden können, die gleichzeitig einen oder mehrere Überlappungsbereiche aufzeichnen.

In bevorzugter Ausführungsform wird bei der Analyse mittels des Analysealgorithmus ein Binärbild erhalten, das mittels einer Hough-Transformation weiter analysiert wird. Aufgrund einer approximativen Herangehensweise durch den Analysealgorithmus können Lücken im Ergebnis der Markierung der Schichtgrenzen enthalten sein. Es ist also unter Umständen mit einer Unterbrechung in der Linienführung einer Schichtgrenze zu rechnen. Diese unterbrochenen Linien, die an mehreren Stellen unterbrochen sein können, werden durch die Hough-Transformation geglättet. Hierbei können mögliche Unterbrechungen dieser Linien geschlossen werden. Durch die Hough-Transformation können die Schichtgrenzen verbessert ermittelt werden.

Besonders bevorzugt wird das Binärbild nach der Analyse mittels der Hough-Transformation anhand eines Clustering-Verfahrens, vorzugsweise eines k-Means-Verfahrens, weiter ausgewertet. Es wird also eine Clusteranalyse durchgeführt. Hierbei werden vorzugsweise alle Punkte, die einem Zentrum zugeordnet werden können und sich am nächsten zueinander in einer Reihe im Binärbild befinden, mit Linien verbunden, um verbleibende Lücken im Binärbild zu schließen und durchgängige Linien zu erhalten. Das Ergebnis der Weiterverarbeitung mittels der Hough-Transformation wird wieder als Binärbild repräsentiert und kann immer noch Lücken in der Linienführung enthalten. Diese Linien sind vorzugsweise im Binärbild mit dem Wert 1 oder weiß markiert. Das heißt, für jeden Pixel des Bilds ist klar festgelegt, ob er einer Linie oder dem Hintergrund zuzuordnen ist. Als Linie ist in diesem Fall insbesondere eine Schichtgrenze zu verstehen. Beispielweise kann wie folgt vorgegangen werden. Um die eventuell verbleibenden Lücken zu schließen, wird zunächst für alle im Bild enthaltenen Punkte mit dem Wert 1 das Zentrum mit der jeweils kürzesten Distanz ermittelt. Alle Punkte, die einem Zentrum zugeordnet werden können und sich am nächsten zueinander in einer Reihe im Bild befinden, werden mit Linien verbunden, sofern sich nicht bereits Punkte mit dem Wert 1 in unmittelbarer Nachbarschaft befinden. Hierdurch können die verbleibenden Lücken geschlossen werden, sodass durchgängige Linien entstehen.

Vorteilhafterweise umfasst die künstliche Intelligenz ein neuronales Netz, das für verschiedene Bohrkernarten basierend auf einer Expertenannotation, von Experten, die zur Asphaltprüfung berechtigt sind, trainiert ist. Die Trainingsdaten für das neuronale Netz sind wenigstens bezüglich eines der folgenden Parameter annotiert: Farbe des Bitumen-Mineralstoffgeschmischs, Korngrößen, Kornverteilung, Porigkeit, Anzahl der Schichten, Kompaktierung der Schichten, Schichtgrenzen, Hohlräume und/oder Farbübergänge zwischen den Schichten. Das neuronale Netz ist für verschiedene Bohrkernarten trainiert. Die Bohrkerne und deren Oberflächen und/oder Bohrkanten unterscheiden sich wenigstens in den oben genannten Parametern. Jede Kombination der oben genannten Parameter ist durch einen entsprechend aufbereiteten Datensatz abgedeckt. Die Datensätze können dabei wie folgt zusammengestellt und für das Training des neuronalen Netzes angepasst sein. Bildaufnahmen von Manteloberflächen verschiedener Bohrkerne jeweils mit Längen 200 mm, 400 mm und 650 mm. Schichtverbünde pro Bohrkern mit zwei, drei, vier und/oder mehr Schichten. Insbesondere werden im Trainingsdatensatz unter anderem folgende Schichtarten unterschieden: Deckschicht, Binderschicht und/oder Tragschicht.

Zusätzlich kann das neuronale Netz auf einen Datensatz trainiert sein, der alle Längen, Schichtverbünde und Merkmale der Bohrkerne enthält. Die Datensätze sind vorzugsweise so zusammengestellt, dass sie jede der oben genannten Kombinationen in einem separaten Datensatz zusammenfassen.

Ferner können die Bilddaten der Datensätze für verschiedene Klassifizierungsverfahren annotiert sein. Die Verfahren können sich wie folgt unterscheiden:
Anmerkung des kompletten Bilds für die Bildklassifizierung. Anmerkung pro Bild für den Bohrkerntyp, beispielsweise bezüglich der Länge, bezüglich der Anzahl der Schichten und fortlaufende Nummerierung von oben, also von der Deckschicht, bis unten, beginnend bei 1. Vorzugsweise ist für jede Schicht der Schichttyp definiert, also ob die Schicht eine Deckschicht, Binderschicht oder Tragschicht ist, wobei der Schichttyp der Nummerierung eindeutig zugeordnet werden kann.

Ferner kann eine Bildsegmentierung und Objekterkennung erfolgt sein. Hierbei kann eine Kennzeichnung eines rechteckigen Bereichs der Schichtgrenzen erfolgen, sodass die Schichtgrenze in ihrer maximalen Ausdehnung im Bild beispielsweise von rechts nach links und mit Ausbrüchen oben und unten umschlossen wird.

Es kann eine pixelweise definierte Linie mit einer beliebigen Anzahl von Punkten von rechts nach links in den Trainingsdaten enthalten sein, die die Schichtgrenze zwischen zwei Schichten markiert.

Es kann eine Zuweisung der Schichtnummer und/oder des Schichttyps zum jeweiligen Rechteck und/oder zur Linie, die die Schichtgrenze markiert, erfolgt sein.

Hierbei kann die maximale Anzahl von Punkten die gesamte Breite des Bilds sein. Dieser Wert kann allerdings stark variieren, da oftmals Polygone mit einer variablen Anzahl an Punkten bei der Annotation eingefügt werden.

Es versteht sich, dass aufgrund beispielsweise normativer Vorgaben, wie beispielsweise in "Technische Prüfvorschriften für Asphalt" beschrieben, mindestens 4 Punkte, analog zur manuellen Vermessung, im Bild annotiert werden müssen.

Normative Vorgaben können sich länderspezifisch oder regional unterscheiden. Es versteht sich, dass die Art und Weise der Annotation den länderspezifischen oder regionalen Vorgaben angepasst sein kann.

Die Annotation oder Anmerkung der Trainingsdaten erfolgt ausschließlich von Experten, die zur Asphaltprüfung berechtigt sind und entsprechend geschult wurden.

Es versteht sich, dass auf Basis der Datensätze mehrere Modelle trainiert werden können, die sich insbesondere darin unterscheiden können, welcher Datensatz und welche Kombination aus den Datensätzen für das Training genutzt wird. Insbesondere können folgende Modelle trainiert und bei der Bildverarbeitung genutzt werden:
Modell zur Erkennung der Schichten unter Verwendung der Datensätze. Hierbei erfolgt ausschließlich eine Erkennung der Schichtgrenze und/oder Trennfläche. Insbesondere können hierbei die Schichten von oben nach unten fortlaufend, beginnend bei 1 nummeriert sein. Oben ist als die oberste Seite des Straßenbelags zu verstehen.

Ein Modell, das der Schichtgrenze den Schichttyp eindeutig zuweist.

Ein Modell, das Poren pixelgenau detektiert, d. h. insbesondere den Umfang und die Größe sowie die Konturen.

Ein Modell, das die Poren und/oder Hohlräume im Rahmen einer Objektdetektion erkennt, also insbesondere ein Rechteck, eine sogenannte Bounding-Box, um die Poren herum erzeugt.

Die Erfindung kann insbesondere als Rollen-Prüfstand zur digitalen fotometrischen Bohrkernaufnahme mit fahrbarem Kamerasystem ausgebildet sein. Dieses System erlaubt die Aufnahme von Bohrkernen über die gesamte Mantelfläche des Bohrkerns mittels vorzugsweise schrittmotorbetriebenem Rollenprüfstand in einer dimmbaren Fotokammer. Die fotogrammametrischen Daten können über einen integrierten PC intern weiterverarbeitet oder alternativ an eine Cloud weitergeleitet werden.

Alle relevanten Prüfdaten, wie Abbildungen der Mantelfläche, eine Abbildung einer Schnittfläche bei einem Schnitt längs mittig durch den Bohrkern, ein Probeentnahmeort, beispielsweise mittels GPS ermittelt, Datum, etc. können automatisiert aufgezeichnet und lokal auf dem PC oder in einer Cloud gespeichert werden.

Der Rollenprüfstand kann insbesondere ein Kamerasystem zur Aufnahme von Bohrkernen bis zu einem Durchmesser von 200 mm sowie einen Rollenstand mit Schrittmotorantrieb aufweisen. Die dimmbare Fotokammer kann mit kippbarer LED-Beleuchtung, vorzugsweise LED-Flächenbeleuchtung, ausgebildet sein, wobei eine künstliche Intelligenz (KI), insbesondere ein neuronales Netz, zum automatischen Analysieren der Bohrkernaufnahmen, vorzugsweise mit einer Lernfunktion, verwendet wird. Es erfolgt ein automatisches Erfassen der Schichtgrenzen für den Schichtverbund des Bohrkerns sowie ein fotogrammetrisches automatisches Ausmessen und virtuelles Einzeichnen der Schichtgrenzen für den erfassten Bohrkern. Zudem kann eine Archivierung der Daten erfolgen. Der Rollenprüfstand kann optional mit einem RFID-Code-Leser ausgestattet sein und einen integrierten Rechner mit einer Tastatur und einem Bildschirm aufweisen.

Es versteht sich, dass weitere prinzipiell im Stand der Technik bekannte Methoden zur Markierung und anschließenden Identifizierung der Bohrkerne, wie insbesondere ein Strichkode, Anwendung finden können.

Insbesondere kann der Rollenprüfstand auf eine Aufstellfläche von ca. 600 x 600 x 2100 mm platziert werden und mit einem elektrischen Anschluss von 230 Volt bei 50-60 Hertz und bei einer Phase betrieben werden. Vorzugsweise ist die Schnittstelle des Rollenprüfstands als Ethernet-Schnittstelle ausgebildet, wobei der Rollenprüfstand ein Gewicht von ca. 70 kg aufweist. Die Kamera bzw. die Kameraeinheit besteht aus einem Gehäuse, das eine Kameraoptik und optional eine Bildverarbeitungseinheit sowie eine Stromversorgungseinheit aufweist. Der Sensor, also die Kamera, ist in der Lage, Aufnahmen mit hoher Auflösung aufzunehmen und an die Bildverarbeitungseinheit weiterzuleiten.

Das Objektfeld erstreckt sich vorzugsweise über drei Stufen über eine maximale Breite von 660 mm. Die Stufen können dabei wie folgt definiert sein:
Stufe 1 ist für Bohrkerne mit einer Länge von 200 mm und einem Durchmesser von 100 mm ausgelegt. Die zweite Stufe ist für Bohrkerne mit einer Länge von 400 mm und einem Durchmesser von 150 mm ausgelegt. Die dritte Stufe ist für Bohrkerne mit einer Länge von 650 mm und einem Durchmesser von 200 mm ausgelegt.

Das Objektfeld erstreckt sich dabei vorzugsweise auf eine Länge von mindestens 100 mm. Die zu vermessenden Bohrkerne weisen vorzugsweise einen Durchmesser von mindestens 100 mm auf. Die Aufnahmen bzw. Bilder werden von der Oberfläche des rotierenden Bohrkerns von mindestens einer Kamera der Kameraeinheit erfasst. Die Erfassung erfolgt dabei über eine Schnittstelle für die industrielle Bildverarbeitung.

Insbesondere können die folgenden Schnittstellen genutzt werden: Gigabit-Ethernet, das Protokoll "GigE-Vison". Die Kamera und ein Netzwerk Interface können über ein Ethernet-Kabel per RJ45-Schnittstelle verbunden sein. Insbesondere kann die USB3Vision-Schnittstelle genutzt werden. Die Kamera ist per USB 3.x über ein Micro-B-zu-USB-A- oder vergleichbares Kabel mit einem USB-Port eines Computers verbunden.

Es kann insbesondere eine der folgenden industriellen Schnittstellen verwendet werden: USB3Vision, GigE-Vision, Camera Link (HS) und/oder CoaXpres.

Optional ist am Computer eine Framegrabber-Karte integriert, mit der die Aufnahmen der Kameraeinheit in den PC übertragen werden. Die Aufnahme erfolgt kontinuierlich softwaregesteuert von der Bildverarbeitungseinheit.

Eine Ausführungsform eines Verfahrens gemäß der vorliegenden Offenbarung kann insbesondere wie folgt kurz beschrieben werden. Es erfolgt ein Bildeinzug, also eine oder mehrere Aufnahmen des Bohrkerns. Anschließend erfolgt ein Zusammenführen der Einzelbilder zu einem durchgängigen Mosaikbild. In diesem Mosaikbild kann eine Detektion der Schichtgrenzen mittels eines Kl-Modells, beispielsweise U-Net, erfolgen, wobei eine Glättung der Linien durch eine Hough-Transformation und im nächsten Schritt eine nächste Nachbarsuche mit k-Means für die korrekte Zuordnung der Punkte zur Schicht erfolgt.

Anschließend erfolgt ein Schließen aller verbleibenden Lücken zwischen den Punkten innerhalb einer Schichtgrenze.

Die Messung erfolgt vorzugsweise auf Basis einer pixelweisen Umrechnung gemäß der optischen Auflösung der Kameraeinheit. Ein Pixel auf den Sensor der Kameraeinheit entspricht einer Auflösung von vorzugsweise näherungsweise 50 µm auf der Objektebene. Ausgehend von der oberen Kante des Bohrkerns wird pro Schicht die Dimension, d. h. die Dicke der Schicht als Abstand der unteren Schichtgrenze zur oberen Kante als Schichtdicke angegeben. Dieser Abstand kann aus den Bilddaten wie folgt ermittelt werden.

Pro Linie wird im Verlauf von links nach rechts im Bild, also in Umfangsrichtung der Mantelfläche des Bohrkerns, der Durchschnitt aller Pixelwerte in y-Richtung, also in axialer Richtung ermittelt. Dieselbe Methode wird für die Stirnkante des Bohrkerns angewandt. Hierbei entstehen vorzugsweise gerade, geglättete Linien, die jeweils die Stirnkante des Bohrkerns und jede Schichtgrenze definieren. Die Distanz, bzw. ein Abstand oder eine Schichtdicke, wird aus dem die jeweilige Linie einschließenden Pixel und kumulierten Pixeln von der Stirnkante bis zur Schichtgrenze ermittelt. Die Berechnung der Distanz erfolgt gemäß der folgenden Berechnungsvorschrift.

Die Schichtdicke in mm entspricht den kumulierten Pixeln zwischen gemittelter Stirnkante und Schichtgrenze mal der Messgenauigkeit des optischen Systems pro Pixel in mm.

In einer alternativen Variante wird die Distanz an einer Vielzahl von Punkten gleich verteilt oder in gleichem Abstand zueinander jeweils zur oberen Kante des Bohrkerns ermittelt.

Mögliche technische Anwendungsgebiete der Vorrichtung umfassen nicht abschließend die folgenden Bereiche: Vermessen von Schichtdicken von Bohrkernen aus Asphalt und Beton und/oder Bewertung von Korngrößen, Bitumengemisch und/oder Porigkeit von Bohrkernen aus Asphalt.

Im Folgenden werden Ausführungsbeispiele der Erfindung unter Bezugnahme auf die beigefügten Abbildungen beschrieben. Es zeigen:
- Figur 1: Eine schematisch vereinfachte Darstellung einer Vorrichtung zum Vermessen eines Bohrkerns;
- Figur 2: schematisch einen Verfahrensschritt des Bildens eines flachen, verzerrungsfreien Abbilds der Manteloberfläche eines Bohrkerns aus Einzelaufnahmen;
- Figur 3: schematisch ermittelte Schichtgrenzen mittels eines Analysealgorithmus;
- Figur 4: schematisch eine Glättung der Schichtgrenzen mittels einer HoughTransformation;
- Figur 5: schematisch das Auffüllen verbleibender Lücken mittels eines k-Means-Verfahrens;
- Figur 6: ein schematisches Flussdiagramm einer automatisierten Bohrkernanalyse;
- Figur 7: eine bevorzugte Ausführungsform einer Vorrichtung zum Vermessen eines Bohrkerns mit einer Vielzahl von Kameras in der Kameraeinheit; und
- Figur 8: schematisch die Schritte eines erfindungsgemäßen Verfahrens.

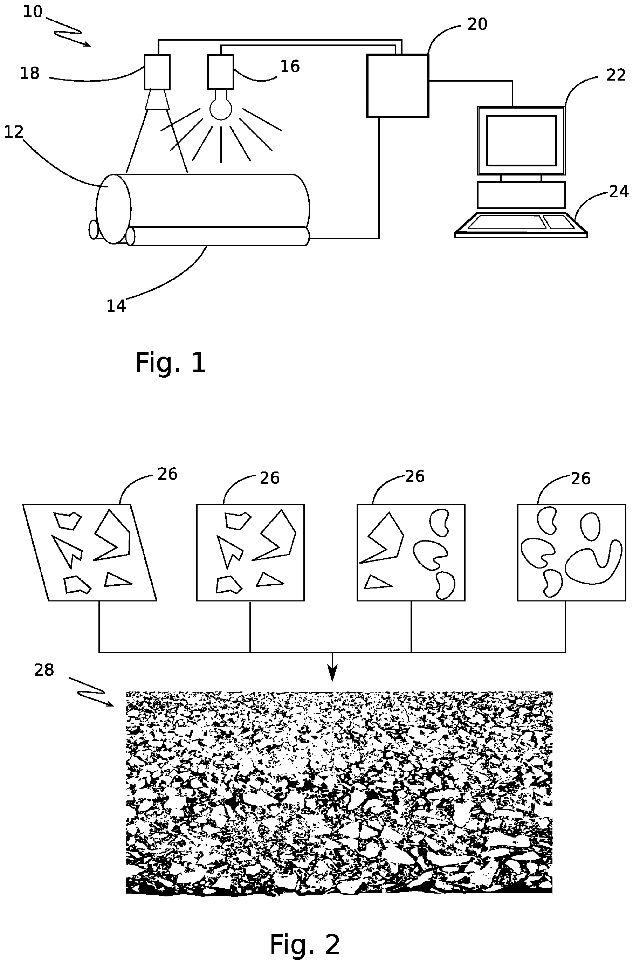

In Figur 1 ist schematisch eine Vorrichtung 10 zum Vermessen eines Bohrkerns 12 eines Straßenbelags dargestellt. Die Vorrichtung 10 weist einen Rollenstand 14 auf, auf dem der Bohrkern 12 gelagert ist. Mittels des Rollenstands 14, der vorzugsweise mit einem Schrittmotorantrieb ausgestattet ist, kann der Bohrkern 12 zur Vermessung um seine Längsachse in Drehung versetzt werden. Der Bohrkern 12 weist im Wesentlichen die Form eines Vollzylinders auf.

Die Vorrichtung 10 umfasst ferner eine Beleuchtungseinheit 16, die dazu ausgebildet ist, den Bohrkern 12 flächig auszuleuchten. Bezüglich der Längsachse des Bohrkerns ist die Beleuchtungseinheit 16 vorzugsweise auf der gleichen Seite wie eine Kameraeinheit 18 angeordnet. Es versteht sich, dass die Beleuchtungseinheit 16 auch in die Kameraeinheit 18 integriert sein kann. Es hat sich jedoch als vorteilhaft erwiesen, diese beiden Einheiten getrennt auszuführen. Durch die Beleuchtungseinheit 16 wird der Bohrkern 12 mit Licht beleuchtet, sodass die Kameraeinheit 18 ein Bild, insbesondere ein Teilbild bzw. eine Teilaufnahme der Manteloberfläche des Bohrkerns 12 erfassen kann.

Die erfassten Aufnahmen der Manteloberfläche des Bohrkerns 12 werden von der Kameraeinheit 18 zu einer Analyseeinheit 20 übertragen. In der Analyseeinheit können dann einzelne Aufnahmen der Manteloberfläche des Bohrkerns 12 zu einem Gesamtbild zusammengefügt werden. Dieses Gesamtbild kann dann einer weiteren automatischen Analyse unterzogen werden, wobei hierbei Schichtgrenzen des Bohrkerns 12 und/oder weitere Parameter wie Farbe des Bitumen-Mineralstoffgemischs, Korngrößen, Kornverteilung, Porigkeit, Anzahl der Schichten, Kompaktierung der Schichten, Hohlräume und/oder Farbübergänge zwischen den Schichten erfasst werden können.

Zur komfortableren Bedienung kann die Analyseeinheit 20 mit einem PC, bzw. Computer 22, mit einer Tastatur 24 und einem Bildschirm verbunden werden. Es versteht sich, dass die Analyseeinheit 20 auch in den Computer 22 integriert sein kann. Die in Figur 1 gewählte Darstellung dient der besseren Übersicht und dem besseren Verständnis der Erfindung.

Zur Verbesserung der Bildqualität kann die Beleuchtungseinheit 16 elektromagnetische Strahlung, insbesondere Licht, verschiedener Wellenlängen und verschiedener Polarisationen emittieren. Es versteht sich, dass die Kameraeinheit 18 zur Erfassung der elektromagnetischen Strahlung ausgebildet ist, und insbesondere für den Fall, dass die Beleuchtungseinheit 16 polarisiertes Licht aussendet, eine entsprechende Optik, insbesondere einen Polarisationsfilter, aufweisen kann.

Figur 2 zeigt schematisch, wie Einzelaufnahmen 26 der Bohrkern-Oberfläche zu einem flachen Gesamtbild 28 der Bohrkern-Oberfläche zusammengeführt werden können. Hierbei ist einerseits eine Verzerrung aufgrund der Krümmung der Oberfläche des Bohrkerns 12 zu korrigieren. Andererseits sind eventuell vorhandene Überlappungsbereiche, also doppelt aufgenommene Bereiche des Bohrkerns 12, zu entfernen. Das Gesamtbild 28 soll im Wesentlichen quasi eine abgerollte Oberfläche bzw. Mantelfläche des Bohrkerns 12 darstellen.

Die Einzelaufnahmen 26 können beispielsweise von einer einzelnen Kamera stammen, die entlang einer Längsachse des Bohrkerns verfahren wird, um den kompletten Bohrkern zu erfassen. Ferner können Einzelaufnahmen 26 aufgrund der Rotation des Bohrkerns bei der Aufnahme entstehen.

Es versteht sich, dass auch mit mehreren Kameras parallel aufgezeichnet werden kann und die so erhaltenen Einzelaufnahmen 26 zu einem Gesamtbild 28 kombiniert werden. Zur Kombination der Einzelaufnahmen können verschiedene Methoden Anwendung finden. Als vorteilhaft hat sich hierbei insbesondere die Anwendung von 3x3 projektiven Transformationsmatrizen erwiesen. Es versteht sich, dass auch andere prinzipiell im Stand der Technik bekannte Verfahren zur Generierung des Gesamtbilds 28 aus den Einzelaufnahmen 26 Anwendung finden können.

Wird der Bohrkern mit wenigstens zwei Kameras erfasst, wobei ein Bereich des Bohrkerns von beiden Kameras gleichzeitig erfasst wird, kann nach dem Stereobildprinzip durch Rektifizierung eine topografische Information der Manteloberfläche berechnet werden. Besonders bevorzugt kann die topografische Information für die komplette Manteloberfläche des Bohrkerns 12 errechnet werden. Hierfür wird vorzugsweise die komplette Manteloberfläche gleichzeitig von beiden Kameras erfasst. Es versteht sich, dass das gleichzeitige Erfassen der kompletten Manteloberfläche schrittweise erfolgen kann. Insbesondere kann das Stereobildprinzip auch mit einer einzigen Kamera, die an vorbekannte Positionen bewegt wird, angewendet werden.

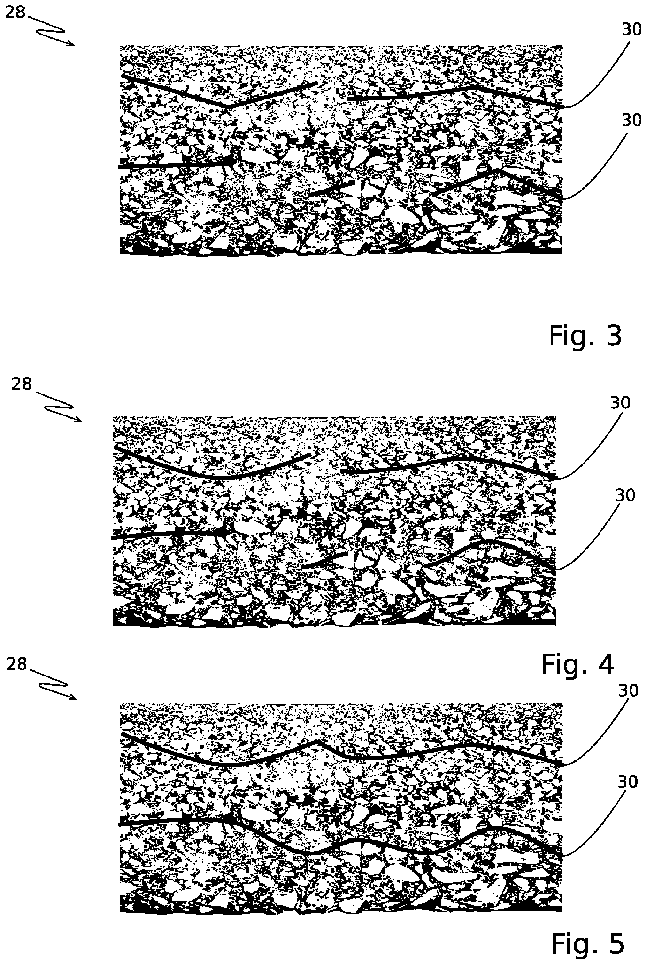

In Figur 3 ist schematisch ein Ergebnis einer Analyse des Gesamtbilds 28 mittels eines Analysealgorithmus in Form eines neuronalen Netzes, das auf einem residualen Netzwerk mit mindestens 18 Schichten basiert, dargestellt. Bei der Analyse mittels des neuronalen Netzes werden Schichtgrenzen 30 der einzelnen Schichten des Bohrkerns markiert. Diese Information wird als Binärbild hinterlegt. Die Schichtgrenzen werden mit einem ersten Wert, beispielsweise 1 markiert und ein Hintergrund mit einem zweiten Wert, beispielsweise 0, markiert. Zur Verdeutlichung ist die Bohrkernaufnahme ebenfalls mit dargestellt. Das Ergebnis der Analyse mittels des neuronalen Netzwerks kann Lücken enthalten, sodass die eingezeichneten Schichtgrenzen 30 potentiell an mehreren Stellen unterbrochen sind.

In Figur 4 ist schematisch das Ergebnis einer Linienglättung mittels einer Hough-Transformation dargestellt. Das Ergebnis des neuronalen Netzes wird mit der sogenannten Hough-Transformation weiterverarbeitet. Hierzu werden die als unterbrochene Linien dargestellten Schichtgrenzen 30 geglättet, sodass mögliche Unterbrechungen in den Schichtgrenzen zumindest teilweise geschlossen werden können.

In Figur 5 ist ein Ergebnis eines weiteren Analyseschritts schematisch dargestellt. Auch nach der Linienglättung durch die Hough-Transformation können noch Lücken in der Linienführung der die Schichtgrenzen 30 anzeigenden Linien enthalten sein. Um die verbleibenden Lücken zu schließen, wird zunächst für alle im Bild enthaltenen Punkte, die mit einem Wert 1 besetzt sind, das Zentrum mit der jeweils kürzesten Distanz ermittelt. Hierbei kommt ein Clustering-Verfahren, insbesondere das Clustering-Verfahren k-Means zum Einsatz. Alle Punkte, die einem Zentrum zugeordnet werden können und sich am nächsten zueinander in einer Reihe im Bild befinden, werden mit Linien verbunden, sofern sich nicht bereits entsprechend markierte Punkte, also Punkte mit dem Wert 1 in unmittelbarer Nachbarschaft befinden. Hierdurch können die verbleibenden Lücken geschlossen werden, sodass durchgängige Linien zur Anzeige der Schichtgrenzen 30 entstehen.

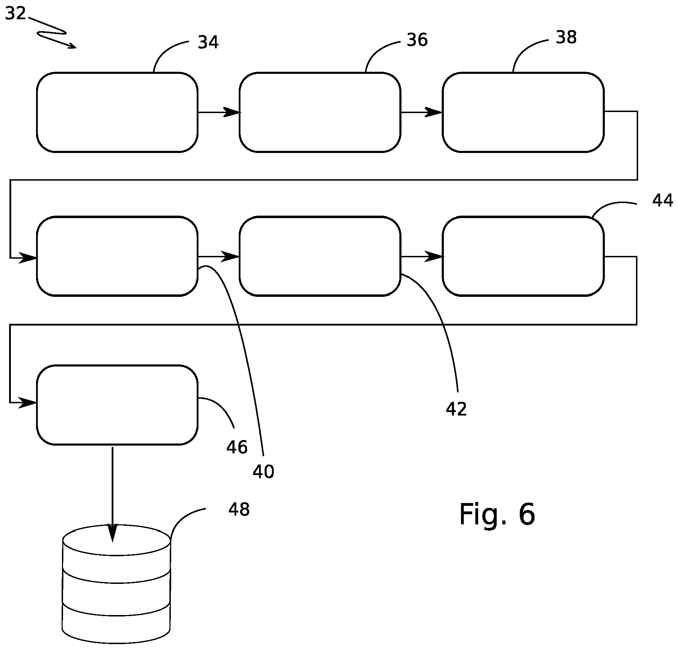

In Figur 6 ist ein Flussdiagramm 32 eines beispielhaften Vermessungsvorgangs, wie er insbesondere mit einer Vorrichtung 10 gemäß Figur 1 durchgeführt werden kann, schematisch dargestellt.

Zu Beginn erfolgt eine Kalibration 34. Im Rahmen der Kalibration 34 kann beispielsweise die Kameraposition relativ zur Objektebene in einem globalen Koordinatensystem berechnet werden. Ein mögliches Kalibrationsverfahren kann per HALCON über Punkt-Target, also Punkt-Ziel, Markierungen und/oder über OpenCV und ein ChArUco-Board erfolgen. Nach dem Kalibrationsverfahren sind die Kameras prinzipiell funktionsfähig und können genutzt werden. Nach einem Bewegen der Kamera kann ein entsprechendes Board wieder unter der Kamera positioniert werden und einmalig gescannt werden, um die neue Position der Kamera zu bestimmen.

Nach der Kalibration 34 kann ein Starten der Rotation 36 des Bohrkerns 12 erfolgen.

Daraufhin erfolgt ein Aufnehmen 38 eines einzelnen oder mehrerer Bilder des in Rotation versetzten Bohrkerns 12.

Anschließend erfolgt ein sogenanntes laterales Stitching 40, bei dem einzelne Aufnahmen von bis zu 7 Kameras lateral zusammengefügt werden.

Daraufhin erfolgt ein horizontales Stitching 42, bei dem die bei der Rotation des Bohrkerns 12 erzeugten Einzelaufnahmen ausgeschnitten und als Abschnitt aneinandergefügt werden.

Daraufhin erfolgt eine Schichterkennung 44, die, wie bereits oben beschrieben, mittels KI und den Folgeanalysen durchgeführt werden kann.

Anschließend kann eine Schichtdickenmessung 46 der einzelnen Schichten des Bohrkerns basierend auf der Schichterkennung erfolgen.

Die Ergebnisse sowie die Aufnahmen und ggf. weitere erfasste Informationen können in einem Speicher, einer Datenbank 48 und/oder einer Cloud hinterlegt werden und so einem breiten Anwenderspektrum zur Verfügung gestellt werden, zum Training weiterer neuronaler Netze verwendet werden, einem Auftraggeber direkt zur Verfügung gestellt werden und/oder weiterverarbeitet werden. Insbesondere ist es denkbar, dass basierend auf der Schichtdickenerkennung automatisiert ein entsprechender Bericht erstellt und übermittelt wird. Es versteht sich, dass weitere darauffolgende Schritte denkbar sind.

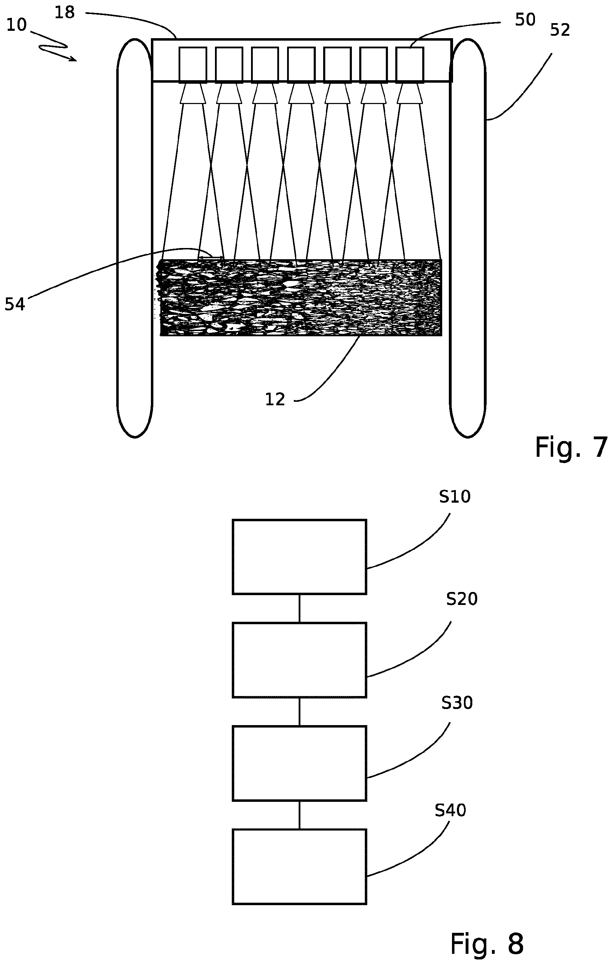

In Figur 7 ist schematisch eine weitere Ausführungsform einer Vorrichtung 10 zum Vermessen eines Bohrkerns 12 gezeigt. Im Unterschied zu der in Figur 1 gezeigten Ausführungsform umfasst die Kameraeinheit 18 in der in Figur 7 gezeigten Ausführungsform sieben Kameras 50. Aus Gründen der Übersicht wurde auf eine Darstellung der Beleuchtungseinheit sowie des Rollenstands verzichtet. Eine Umhausung 52, die zum Ausbilden einer Fotokammer dienen kann, ist vereinfacht schematisch dargestellt.

Die sieben einzelnen Kameras 50 der Kameraeinheit 18 erfassen den Bohrkern 12 und sind derart angeordnet, dass beim Erfassen einzelner Bohrkernabschnitte ein Überlappungsbereich 54 erfasst wird. Das heißt, es sind Bereiche auf der Manteloberfläche des Bohrkerns 12 vorhanden, die von wenigstens zwei Kameras gleichzeitig erfasst werden. Beispielsweise kann ein Überlappen von 30% pro Kamera 50 vorgesehen sein.

Durch das Vorsehen von bis zu sieben oder mehr Kameras 50 kann ein Bohrkern schneller vermessen werden. Zudem ermöglicht die Aufnahme eines Überlappungsbereichs 54 das Ermitteln von topografischen Informationen der Bohrkern-Oberfläche, wie bereits oben beschrieben.

In Figur 8 sind die wesentlichen Schritte eines erfindungsgemäßen Verfahrens zur Vermessung eines Bohrkerns eines Straßenbelags schematisch dargestellt.

In einem ersten Schritt S10 erfolgt ein in Drehung Versetzen des Bohrkerns. Das in Drehung Versetzen S10 des Bohrkerns erfolgt vorzugsweise über einen Rollenstand. Es versteht sich, dass auch andere Verfahren hierfür denkbar sind.

In einem zweiten Schritt S20 erfolgt ein Beleuchten des Bohrkerns mit einer Beleuchtungseinheit. Die Beleuchtungseinheit kann insbesondere als flächiges LED-Panel, besonders bevorzugt als zwei flächige LED-Panels ausgebildet sein, wobei sich die beiden flächigen LED-Panels parallel zu einer Längsachse des Bohrkerns erstrecken und besonders bevorzugt schräg zueinander symmetrisch um die Längsachse des Bohrkerns angeordnet sind. Besonders bevorzugt ist die Kameraeinheit zwischen diesen beiden Flächenlichtern angeordnet.

In einem weiteren Schritt S30 erfolgt ein Aufnehmen des in Drehung versetzten und beleuchteten Bohrkerns mit der Kameraeinheit. Hierbei können insbesondere Einzelbilder von Bohrkern-Abschnitten aufgenommen werden.

Anschließend wird in einem vierten Schritt S40 der aufgenommene Bohrkern bezüglich seiner Beschaffenheit analysiert, wobei der Bohrkern mittels eines Analysealgorithmus automatisch bezüglich wenigstens eines der folgenden Parameter analysiert wird: Farbe des Bitumen-Mineralstoffgemischs, Korngrößen,

Kornverteilung, Porigkeit, Anzahl der Schichten, Kompaktierung der Schichten, Schichtgrenzen, Hohlräume und/oder Farbübergänge zwischen den Schichten.

Es versteht sich, dass im Rahmen der Analyse vorzugsweise die im dritten Schritt S30 aufgenommenen Einzelbilder zu einem Gesamtbild zusammengefügt werden. Es ist jedoch auch denkbar, die vorgenannten Parameter anhand einer einzelnen Komplettaufnahme des Bohrkerns zu bestimmen.

Die Erfindung wurde anhand der Zeichnungen und der Beschreibung umfassend beschrieben und erklärt. Die Beschreibung und Erklärung sind als Beispiel und nicht einschränkend zu verstehen. Die Erfindung ist nicht auf die offenbarten Ausführungsformen beschränkt. Andere Ausführungsformen oder Variationen ergeben sich für den Fachmann bei der Verwendung der vorliegenden Erfindung sowie bei einer genauen Analyse der Zeichnungen, der Offenbarung und der nachfolgenden Patentansprüche.

In den Patentansprüchen schließen die Wörter "umfassen" und "mit" nicht das Vorhandensein weiterer Elemente oder Schritte aus. Der undefinierte Artikel "ein" oder "eine" schließt nicht das Vorhandensein einer Mehrzahl aus. Ein einzelnes Element oder eine einzelne Einheit kann die Funktionen mehrerer der in den Patentansprüchen genannten Einheiten ausführen. Ein Element, eine Einheit, eine Schnittstelle, eine Vorrichtung und ein System können teilweise oder vollständig in Hard- und/oder in Software umgesetzt sein. Die bloße Nennung einiger Maßnahmen in mehreren verschiedenen abhängigen Patentansprüchen ist nicht dahingehend zu verstehen, dass eine Kombination dieser Maßnahmen nicht ebenfalls vorteilhaft verwendet werden kann. Ein Computerprogramm bzw. ein Computerprogrammprodukt kann auf einem nichtflüchtigen Datenträger gespeichert/vertrieben werden, beispielsweise auf einem optischen Speicher oder auf einem Halbleiterlaufwerk (SSD). Ein Computerprogramm kann zusammen mit Hardware und/oder als Teil einer Hardware vertrieben werden, beispielsweise mittels des Internets oder mittels drahtgebundener oder drahtloser Kommunikationssysteme. Bezugszeichen in den Patentansprüchen sind nicht einschränkend zu verstehen.

### Bezugszeichenliste

- 10: Vorrichtung
- 12: Bohrkern
- 14: Rollenstand
- 16: Beleuchtungseinheit
- 18: Kameraeinheit
- 20: Analyseeinheit
- 22: Computer
- 24: Tastatur
- 26: Einzelaufnahmen
- 28: Gesamtbild
- 30: Schichtgrenzen
- 32: Flussdiagramm
- 34: Kalibration
- 36: Starten der Rotation
- 38: Aufnehmen von Bildern des Bohrkerns
- 40: Laterales Stitching
- 42: Horizontales Stitching
- 44: Schichterkennung
- 46: Schichtdickenmessung
- 48: Datenbank
- 50: Kamera
- 52: Umhausung
- 54: Überlappungsbereich
- S10 - S40: Schritte eines erfindungsgemäßen Verfahrens

## Patentansprüche

1. Verfahren zum Vermessen eines Bohrkerns (12) eines Straßenbelags umfassend die Schritte:
In Drehung Versetzen (S10) des Bohrkerns (12);
Beleuchten (S20) des Bohrkerns (12) mit einer Beleuchtungseinheit (16);
Aufnehmen (S30) des in Drehhung versetzten und beleuchteten Bohrkerns (12) mit einer Kameraeinheit (18); und
Analysieren (S40) des aufgenommenen Bohrkerns (12) bezüglich seiner Beschaffenheit, wobei der Bohrkern (12) mittels eines Analysealgorithmus automatisiert bezüglich eines der folgenden Parameter analysiert wird: Farbe des Bitumen-Mineralstoffgemischs, Korngrößen, Kornverteilung, Porigkeit, Anzahl der Schichten, Kompaktierung der Schichten, Schichtgrenzen (30), Hohlräume und/oder Farbübergänge zwischen den Schichten.

2. Verfahren nach dem vorstehenden Anspruch, wobei der Analysealgorithmus eine künstliche Intelligenz, bevorzugt ein neuronales Netz, das besonders bevorzugt auf einem residualen Netzwerk basiert, umfasst.

3. Verfahren nach einem der vorstehen Ansprüche, wobei das Beleuchten (S20) des Bohrkerns (12) mittels einer Beleuchtungseinheit (16) mit zwei Flächenlichtern, die parallel zur Lage des Bohrkerns (12) angebracht sind, durchgeführt wird.

4. Verfahren nach einem der vorstehenden Ansprüche, wobei der Bohrkern (12) mit weißem Licht beleuchtet wird;
vorzugsweise mit rotem, blauem und/oder grünem Licht abwechselnd beleuchtet wird; und/oder
besonders bevorzugt mit polarisiertem Licht beleuchtet wird.

5. Verfahren nach einem der vorstehenden Ansprüche, mit den Schritten: Entzerren und Zuschneiden von Einzelaufnahmen (26) des Bohrkerns (12) durch Anwendung von Transformationsmatrizen, sodass zwischen den Einzelaufnahmen (26) von Bohrkernabschnitten keine Überlappungsbereiche (54) mehr sichtbar sind und ein flaches, entzerrtes Gesamtbild (28) der Mantelfläche des Bohrkerns (12) geschaffen wird.

6. Verfahren nach einem der vorstehenden Ansprüche, wobei ein Überlappungsbereich (54) erfasst wird, und basierend auf der Erfassung des Überlappungsbereichs (54) eine topographische Information des Bohrkerns (12) bestimmt wird.

7. Verfahren nach einem der vorstehenden Ansprüche, wobei bei der Analyse mittels des Analysealgorithmus ein Binärbild erhalten wird, das mittels einer Hough-Transformation weiter analysiert wird.

8. Verfahren nach dem vorstehenden Anspruch, wobei das Binärbild nach der Analyse mittels der Hough-Transformation anhand eines Clustering-Verfahrens, vorzugsweise eines k-Means Verfahrens, weiter ausgewertet wird, wobei vorzugsweise alle Punkte, die einem Zentrum zugeordnet werden können und sich am nächsten zueinander in einer Reihe im Binärbild befinden mit Linien verbunden werden, um verbleibende Lücken im Binärbild zu schließen und durchgängige Linien zu erhalten.

9. Verfahren nach einem der Ansprüche 2 bis 8, wobei die künstliche Intelligenz ein neuronales Netz, das für verschiedene Bohrkernarten basierend auf einer Experten-Annotation trainiert ist, umfasst, wobei die Trainingsdaten für das neuronale Netz wenigstens bezüglich eines der folgenden Parameter annotiert sind: Farbe des Bitumen-Mineralstoffgemischs, Korngrößen, Kornverteilung, Porigkeit, Anzahl der Schichten, Kompaktierung der Schichten, Schichtgrenzen (30), Hohlräume und/oder Farbübergänge zwischen den Schichten.

10. Vorrichtung (10) zum Vermessen eines Bohrkerns (12) eines Straßenbelags umfassend:
einen Rollenstand (14) zum in Drehung Versetzen des Bohrkerns (12);
eine Beleuchtungseinheit (16) zum Beleuchten des Bohrkerns (12);
eine Kameraeinheit (18) zum Aufnehmen des in Drehung versetzten und beleuchteten Bohrkerns (12); und
eine Analyseeinheit (20), die dazu ausgebildet ist, Schichtgrenzen (30) des Bohrkerns (12) automatisiert zu erfassen, und vorzugsweise ein Verfahren nach einem der vorstehenden Ansprüche durchzuführen.
